# EUROPEAN PATENT APPLICATION

(11) **EP 2 589 394 A1**
(43) Date of publication of application: **08.05.2013**
(21) Application number: 11191571.6
(22) Date of filing: 01.12.2011
(51) Int. Cl.: A61M 1/00

(54) **Integrated blood donor tube stripper and crimping apparatus**

(30) Priority: 01.11.2011 US 201113317895
(71) Applicant: Melco Engineering Corporation, Los Angeles, CA 91372 (US)
(72) Inventor: David, Henry B., California 91372 (US)
(74) Representative: Coulon, Ludivine

(57) **Abstract**

A pair of gripping frame members (11,12) are hingedly connected together at one end thereof. Cooperating rollers (16,17) are rotatably coupled to the gripping members (11,12) adjacent the hinge (13,14) therebetween. The rollers (16,17) are positioned adjacent one another whereby a uniform space is created between the parallel, aligned surfaces of the rollers (16,17) which are adapted to receive flexible fluid-filled tubing (15) connected to a blood donor bag. When the gripping frame members (11,12) are positioned adjacent one another, the aligned surfaces of the rollers (16,17) will fully compress the surfaces of the tubing (15). Opposed crimping heads (30,31) coupled to the gripping frame members (11,12) are adapted to crush a securing clip (34) disposed about the tubing (15) thereby sealing the fluid-filled tubing (15) when the heads (30,31) are positioned adjacent one another.

## Description

### Background of the Invention.

### 1. Field of the Invention.

The present invention generally relates to apparatus used to strip fluid from flexible plastic tubing and in particular to strip blood from tubing incorporated with a blood donor bag.

### 2. Description of the Prior Art.

The prior art discloses system used to collect blood from a donor.

The most common apparatus uses a plastic blood collecting and dispensing bag having flexible plastic tubing integral therewith which communicates with the interior of the bag. The integral flexible plastic tubing is provided with a hollow needle which is inserted into the vein of the donor thereby allowing blood to flow from the donor, through the interior of the plastic tubing and into the blood collecting and dispensing bag. After the blood is collected, the prior art discloses sealing the tube by means of a knot formed therein after which the needle is removed and discarded.

Prior to use of the collecting and dispensing bag, the bag is filled with an anticoagulant solution to prevent the donated blood from clotting or otherwise congeal. However, after filling the collecting and dispensing bag, the tubing between the needle and the bag will be filled with blood. Since the blood in the tubing has not been mixed with the anticoagulant solution in the bag, blood must be substantially stripped or removed from the tubing and mixed with the contents of the bag in order to prevent coagulation of blood in the tubing. The prior art does disclose apparatus for partially stripping the blood from the tubing, it is ineffective since it allows the blood to flow back into the tubing and, in many instances, sufficient unmixed blood remains in the tubing which will coagulate.

The present invention resolves the problems created by the inadequacies in the devices taught by the prior art. An objective of the present invention is to cause all blood in the tubing to be moved into the collecting and dispensing bag. To accomplish this objective, cooperating rollers are rotatably coupled to each of the hinged gripping frame members. A uniform gap is created between the roller surfaces thereof and, when urged along the tubing toward the collecting and dispensing bag, all blood within the interior passage of the tubing will be forced into the bag and thereby be mixed with the anticoagulating solution A pair of cooperating crimping heads are secured to the gripping frame members. The crimping heads that are adapted to crush sealing clips that are disposed about a segment of the tubing that has been purged of blood. After the crimping heads are activated and the sealing clip is crushed about the tubing closing the interior passage of the tubing, the outflow of blood will be precluded.

### Summary of the Invention.

The present invention provides an improved apparatus for substantially stripping fluids from a flexible plastic tubing used to transfer donated blood from a donor to a collecting and dispensing bag and sealing the tubing thereafter. A pair of gripping frame members are hingedly connected at one end thereof. A cylindrical roller is rotatably coupled to each of the gripping frame members adjacent the hinged end and are adapted to be cooperatively engaged with one another when the gripping frame members are positioned adjacent one another. The lateral edges and the cylindrical, perpendicular surface of each roller will, when engaged, define a restricted space that is less than the compressed thickness of the tubing from which blood is to be stripped. To strip the blood from the tubing, the tubing is placed between the peripheral surfaces of the rollers and the hinged gripping frame members placed adjacent one another. Once the tubing is compressed between the peripheral surfaces of the rollers, the present invention is urged toward the coupling between the tubing and the collecting and dispensing bag thereby stripping the blood from the tubing and forcing all blood in the tubing into the collecting and dispensing bag where it will be mixed with the anticoagulant solution.

In order to close off the tubing, crimping heads are secured to the gripping frame members in axial alignment with the axis of the roller coupled to the gripping frame member. The tubing can be sealed by deforming a metallic sealing ring about the tubing. The gripping heads include cooperating crimping slots between which the sealing ring is placed. When the gripping frame members are positioned adjacent one another, the sealing ring will be deformed closing off the interior of the plastic tube thereby precluding egress of blood from the collecting and dispensing bag and attached tubing.

It is therefore an object of the present invention to provide an improved integrated apparatus for stripping a fluid from a flexible tube and sealing the tube.

It is another object of the present invention to provide an integrated apparatus that permits the stripping of fluid from a plastic tube and sealing the tube.

It is still another object of the present invention to provide an improved apparatus for substantially and completely crimping blood from a donor tube for mixing with an anticoagulant solution.

It is still yet another object of the present invention to provide an integrated blood donor, tube stripper apparatus and crimping apparatus that is simple and inexpensive to fabricate.

The novel features which are believed to be characteristic of the invention, both as to its organization and method of operation, together with further objectives and advantages thereof, will be better understood from the following description considered in connection with the accompanying drawing in which a presently preferred embodiment of the invention is illustrated by way of example. It is to be expressly understood, however, that the drawing is for the purpose of illustration and description only, and is not intended as a definition of the limits of the invention.

### Brief Description of the Drawing.

FIGURE 1 is a perspective view of the present invention blood donor tube stripper and crimping apparatus illustrating the positioning of the stripping rollers.
FIGURE 2 is a perspective view of the present invention blood donor tube stripper and crimping apparatus illustrating the gripping heads sealing member in detail.
FIGURE 3 is a side elevation view showing the gripping frame members in an open position in relation to the placement of the tubing from which blood is to be stripped.
FIGURE 4 is a side elevation view of the present invention illustrating the positioning of the tubing to be stripped between the cooperating rollers
FIGURE 5 is a cross-sectional view of the alignment members extending from the gripping frame members taken through line 5-5 of FIGURE 4.
FIGURE 6 is a cross-sectional view of the gripping frame members and crimping heads taken through line 6-6 of FIGURE 4.
FIGURE 7 is a partial, side elevation view of a segment of a donor tube and a sealing clip.
FIGURE 8(a) is a partial, side elevation view of the crimping heads shown in FIGURE 2.
FIGURE 8(b) is a partial, side elevation view of the crimping heads shown in FIGURE 8(a) with a sealing clip positioned between the gripping heads.

### Description of the Presently Preferred Embodiment.

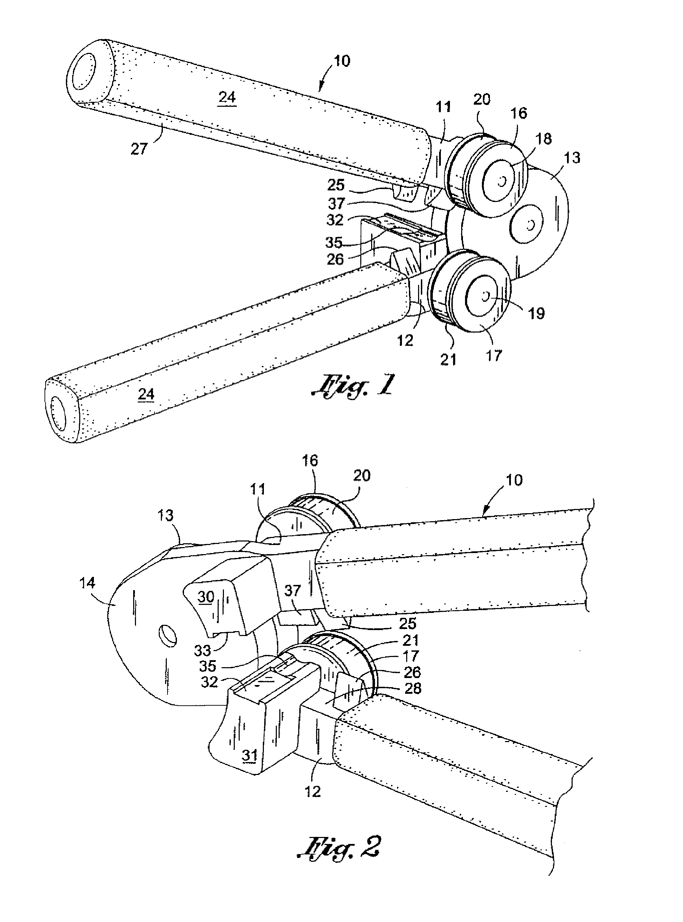

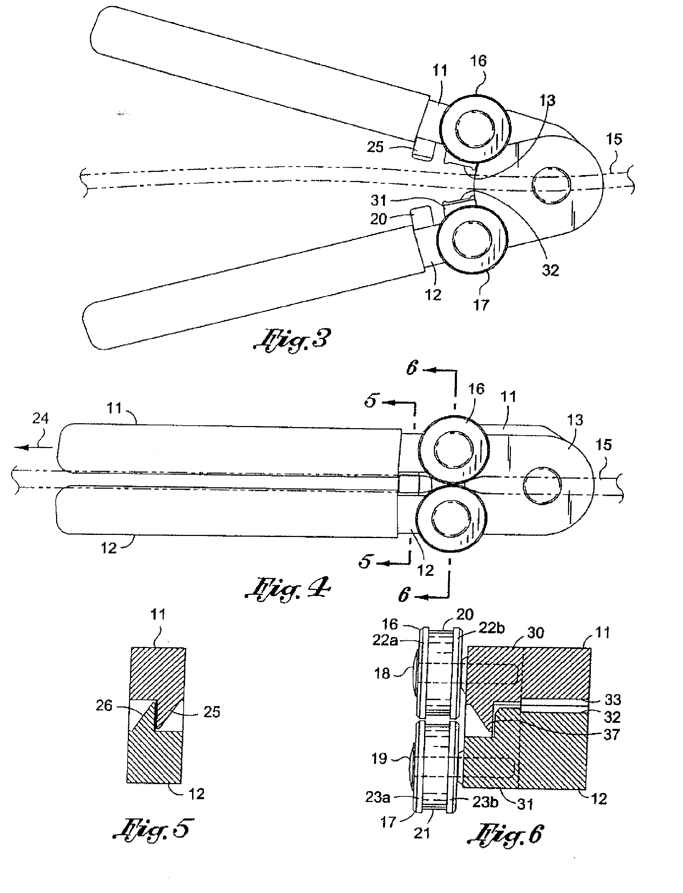

The present invention can be best understood by reference to FIGURES 1 and 2 which illustrate perspective views of the present invention generally designated by the reference numeral 10. The present invention comprises a pair of gripping frame members 11 and 12 that are hingedly coupled to one another by interlocking pivoting couplings 13 and 14 respectively. To interlock pivotal couplings 13 and 14, the preferred embodiment preferably employs a conventional needle bearing assembly which will distribute the imposed load and reduce friction. Although it is understood that the use of a needle bearing assembly is preferable, it is understood that other conventional ball or roller bearings can be used in lieu thereof. The interior cavities of pivoting couplings 13 and 14 (not shown) also includes conventional spring biasing that maintain the quiescent open position of gripping frame members shown in FIGURES 1 and 2 and which applies resilient force to gripping frame members 11 and 12 when they are positioned adjacent one another as shown in FIGURE 4. The quiescent position of gripping frame members 11 and 12 allows the positioning of donor tube 15 between stripping rollers 16 and 17.

As shown in FIGURE 6, gripping frame members 11 and 12 are provided with cylindrical rod members 18 and 19, respectively, the ends of which extend into apertures disposed in gripping frame members 11 and 12, respectively, so as to be securely mounted therein. Stripping rollers 16 and 17 are rotatably mounted on rods 18 and 19, respectively, and easily rotate thereon. Each stripping roller 16 and 17 consists of a cylindrical peripheral surface 20 and 21, respectively. Peripheral surfaces 20 and 21 are bounded by tubing guides 22a, 22b, and 23a, 23b, respectively. As can be best seen in FIGURES 3 and 6, the rod members 18 and 19 and stripping rollers 16 and 17 carried thereby provide for the peripheral surfaces 20 and 21 to be spaced apart a distance whereby the portion of the fluid filled tubing 15 which is passing between the peripheral surfaces 20 and 21 is compressed to a thickness less than normal wall thickness, whereby the fluid may be substantially completely stripped from donor tubing 15.

It is an objective of the present invention to provide an improved apparatus for substantially and completely stripping blood from a donor tube for mixing with an anticoagulant solution. As shown best in FIGURE 6, it is essential that stripping rollers 16 and 17 be properly aligned when gripping rollers are moved to the position shown in FIGURE 4. The proper position would result in alignment of tubing guides 22 and 23 and peripheral surfaces 20 and 21 of stripping rollers 16 and 17. To prevent the inadvertent movement of gripping frame members 11 and 12 during the alignment of stripping rollers 16 and 17 about donor tube 15, the surfaces of gripping frame members 11 and 12 have disposed thereon a covering layer 24 having a sticky gripping surface or otherwise have a low durometer rating (i.e., soft), as that term is understood in the trade.

The manner in which stripping rollers 16 and 17 will be properly aligned may be best seen by reference to FIGURES 1, 2 and 5. To insure proper alignment of gripping frame members 11 and 12 and stripping rollers 16 and 17, alignment flanges 25 and 26 are provided. Alignment flange 25 is secured to the bottom surface 27 of gripping frame member 11. To provide the cooperative relationship between alignment flanges 25 and 26 as shown in FIGURE 5, alignment flange 26 is secured to the upper surface 28 of gripping frame member 12 at a place where the adjacent perpendicular surfaces of alignment flanges 25 and 26 will be slidably engaged with one another. In operation, when gripping frame members 11 and 12 are urged into the position shown in FIGURE 4, alignment flanges 25 and 26 will be slidably engaged as shown in FIGURE 5 and stripping rollers 16 and 17 properly aligned as shown in FIGURE 6 and to prevent the inadvertent dislodgement of donor tube 15 from between stripping rollers 16 and 17.

To strip blood from donor tube 15, gripping frame members 11 and 12 are urged together as shown in FIGURE 4 compressing donor tube 15 between peripheral surfaces 20 and 21 of stripping rollers 16 and 17, respectively. Since the space between peripheral surfaces 21 and 22 will result in the compression of the wall of the donor tubing, when the present invention 10 is urged along the axis of donor tube 15 toward the collecting and disbursing bag (not shown) in a manner that is schematically depicted by directional reference 24, the blood that had been contained in the tubing between the closure of stripping rollers 16 and 17 and the bag will be forced into the bag for mixing with the anticoagulant solution.

The prior art generally discloses the folding of the tube upon itself as a temporary means for preventing the egress of blood in the tubing that has not been mixed with the anticoagulant solution. The present invention substantially improves upon the prior art through the use of the cooperating crimping heads 30 and 31 illustrated in FIGURES 1, 2, 6 and 8. Gripping head 30 is secured to gripping frame member 11 in axial alignment with rod member 18 and stripping roller 16. Gripping head 31 is secured to gripping frame member 12 in axial alignment with rod member 19 and stripping roller 17. Stripping roller 31 includes on the cooperating upper surface thereof a uniform depression 32 on which donor tube 15 will be placed. The cooperating lower surface of gripping head 30 has disposed therein a uniform depression 33 that is coextensive with depression 32 in gripping head 31.

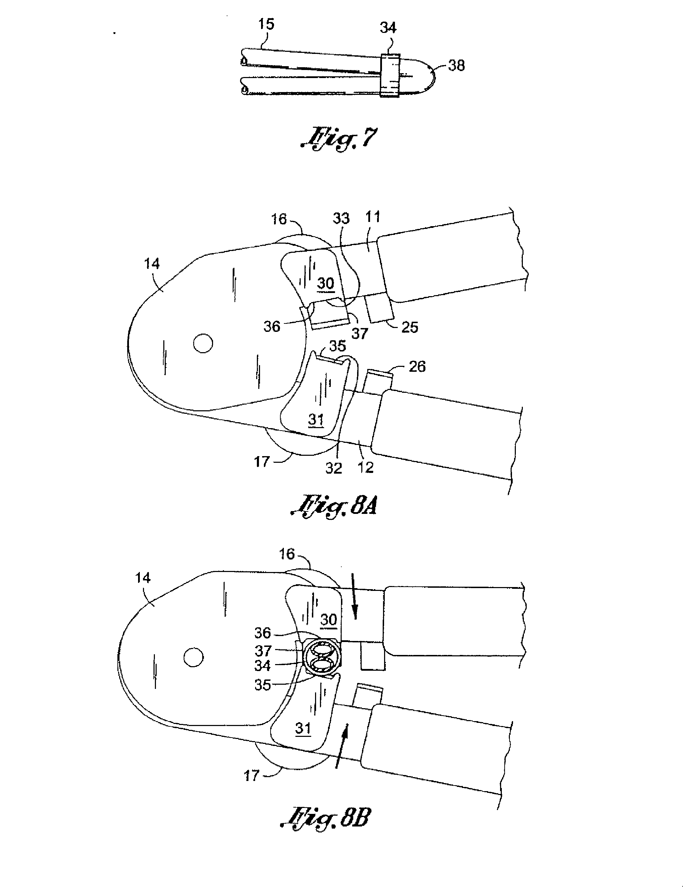

It is an object of the present invention to provide means to seal donor tube 15 with a sealing clip 34. As shown in FIGURE 7, donor tube 15 is folded upon itself to close off the interior passage in which blood will be disposed. Sealing clip 34 is snugly mounted upon the exterior surface of donor tubing 15. It is understood that conventional rectangular, round or oval seals can be employed with the present invention. As shown in FIGURE 2 and FIGURES 8(a) and 8(b), stamping depression 35 is disposed in the upper surface of gripping head 31 adjacent tube depression 32 in a form receptive to the configuration of sealing clip 34 [FIGURE 8(b)]. As can be seen in FIGURE 8(b), stamping depression 36 is disposed in the surface of gripping head 30 to secure sealing clip 34 in position. In operation, when the donor tube 15 and mounted sealing clip 34 is positioned between crimping heads 30 and 31 and gripping frame members 11 and 12 positioned as shown in FIGURE 4. The forces imposed by sealing depressions 35 and 36 on sealing clip 34 will crush sealing clip 34 thereby closing the interior passage of donor tube 15. Tube trimming flange 37 extends from gripping frame member 11 immediately adjacent stamping depression 35. When the gripping heads are fully closed and sealing clip 34 crushed, the folded portion 38 of donor tube 15 extending from sealing clip 34 will be severed by tube trimming flange 37. The present invention thereby provides an integrated blood donor tube stripper and crimping apparatus that substantially overcomes all deficiencies exhibited by the prior art.

## Claims

1. An apparatus for stripping fluid from fluid-filled tubing and sealing same comprising:
(a) first and second linear gripping members being pivotally coupled at one end thereof;
(b) first and second stripping rollers, each having a uniform cylindrical surface and tubing guides being secured to the opposed ends of the cylindrical surface of said first and second stripping rollers in axial alignment with the axis thereof, said first and second stripping rollers being rotatably coupled to said first and second gripping members respectively, whereby the cylindrical surfaces of said stripping rollers are adapted to define a uniform gap therebetween for receipt of the plastic tubing when said first and second gripping members are pivotally rotated adjacent one another;
(c) a first crimping head secured to said first gripping member in alignment with the axis of said first stripping roller incorporating a first stamping surface extending downwardly from said first crimping head and being aligned in parallel spaced relation to the axis of the pivotal coupling between said first and second gripping members; and
(d) a second crimping head secured to said second gripping member in alignment with the axis of said second stripping roller incorporating a second stamping surface extending upwardly from said second crimping head and aligned in parallel spaced relation to the pivotal coupling between said first and second gripping members, said second stamping surface being adapted to be positioned adjacent said first stripping surface when said first and second gripping members are pivotally rotated adjacent one another.

2. An apparatus for stripping fluid from fluid-filled tubing and sealing same as defined in Claim 1 including a first alignment flange having an alignment surface depending downwardly from said first gripping member and a second alignment flange having an alignment surface extending upwardly from said second gripping member, the alignment surface of said second alignment flange being adapted to slidably engage the alignment surface of said first alignment flange when said first and second gripping members are pivotally rotated adjacent one another.

3. An apparatus for stripping fluid from fluid-filled tubing and sealing same as defined in Claim 2 wherein the alignment surfaces of said first and second alignment flanges are coplanar with the plane of said coupled first and second gripping members.

4. An apparatus for stripping fluid from fluid-filled tubing and sealing same as defined in Claim 1 wherein the pivotal coupling between said first and second linear gripping members include resilient means for resiliently opposing the rotation of said first and second gripping members toward one another.

5. An apparatus for stripping fluid from fluid-filled tubing and sealing same as defined in Claim 1 wherein said first and second stamping surfaces of said first and second crimping heads each include opposed depressions adapted to receive a sealing member therebetween.

6. An apparatus for stripping fluid from fluid-filled tubing and sealing same as defined in Claim 1 wherein the cylindrical surfaces of said first and second stripping rollers are in parallel spaced relation when said first and second gripping members are pivotally rotated adjacent one another.

7. An apparatus for stripping fluid from fluid-filled tubing and sealing same as defined in Claim 6 wherein the tubing guides of each of said first and second stripping rollers are in parallel spaced relation to each other and are coplanar with the tubing guides of the opposed stripping roller.

8. An apparatus for stripping fluid from fluid-filled tubing and sealing same comprising:
(a) first and second linear gripping members;
(b) means for resiliently and pivotally coupling an end of each of said first and second gripping members to one another to resiliently oppose the rotation of said first and second gripping members toward one another;
(c) first and second stripping rollers, each having a uniform cylindrical surface and tubing guides being secured to the opposed ends of the cylindrical surface of said first and second stripping rollers in axial alignment with the axis thereof, said first and second stripping rollers being rotatably coupled to said first and second gripping members respectively, whereby the cylindrical surfaces of said stripping rollers are adapted to define a uniform gap therebetween for receipt of the plastic tubing when said first and second gripping members are pivotally rotated adjacent one another;
(d) a first crimping head secured to said first gripping member in alignment with the axis of said first stripping roller incorporating a first stamping surface extending downwardly from said first crimping head and being aligned in parallel spaced relation to the axis of the pivotal coupling between said first and second gripping members;
(e) a second crimping head secured to said second gripping member in alignment with the axis of said second stripping roller incorporating a second stamping surface extending upwardly from said second crimping head and aligned in parallel spaced relation to the pivotal coupling between said first and second gripping members, said second stamping surface being adapted to be positioned adjacent said first stripping surface when said first and second gripping members are pivotally rotated adjacent one another, the first and second stamping surfaces of said first and second crimping heads each including opposed depressions adapted to receive a tube sealing member therebetween;
(f) a first alignment flange having an alignment surface depending downwardly from said first gripping member; and
(g) a second alignment flange having an alignment surface extending upwardly from said second gripping member, the alignment surface of said second alignment flange being adapted to slidably engage the alignment surface of said first alignment flange when said first and second gripping members are pivotally rotated adjacent one another.

9. An apparatus for stripping fluid from fluid-filled tubing and sealing same as defined in Claim 8 wherein the alignment surfaces of said first and second alignment flanges are coplanar with the plane of said coupled first and second gripping members.

10. An apparatus for stripping fluid from fluid-filled tubing and sealing same as defined in Claim 8 wherein the cylindrical surfaces of said first and second stripping rollers are in parallel spaced relation when said first and second gripping members are pivotally rotated adjacent one another.

11. An apparatus for stripping fluid from fluid-filled tubing and sealing same as defined in Claim 8 wherein the tubing guides of each of said first and second stripping rollers are in parallel spaced relation to each other and are coplanar with the tubing guides of the opposed stripping roller.
